# FASCICULE DE BREVET EUROPEEN

(11) **EP 2 124 961 B1**
(45) Date de publication et mention de la délivrance du brevet: **15.01.2014**
(21) Numéro de dépôt: 08787851.8
(22) Date de dépôt: 26.03.2008
(51) Int. Cl.: A61K 31/575, A61K 9/08, A61K 9/10, A61K 47/44, A61K 36/00, A61P 25/28

(54) **NOUVELLE COMPOSITION À BASE D'OXIME DE CHOLEST-4-ÈN-3-ONE**
NEUARTIGE ZUSAMMENSETZUNG AUF DER BASIS VON CHOLEST-4-IN-3-ON-OXIM
NOVEL COMPOSITION BASED ON CHOLEST-4-ENE-3-ONE OXIME

(30) Priorité: 28.03.2007 FR 0702247
(43) Date de publication de la demande: 02.12.2009
(73) Titulaire: Trophos, 13288 Marseille Cedex 9 (FR)
(72) Inventeur: DROUOT, Cyrille, F-83300 Draguignan (FR); BERNA, Patrick, 13260 Cassis (FR)
(74) Mandataire: Galup, Cédric Olivier Nicolas
(86) Numéro de dépôt international: PCT/FR2008/000406
(87) Numéro de publication internationale: WO 2008/142231

(56) Documents cités:
- WO-A-2004/082581
- UNIQEMA: "Drug delivery systems for poorly water-soluble drugs" PDF DOCUMENT CAPABILITY BROCHURES, [Online] 14 décembre 2005 (2005-12-14), pages 1-2, XP002457168 Extrait de l'Internet: URL:http://www.uniqema.com/irj/servlet/prt /portal/prtroot/com.uniqema.portal.Product DetailsProject.ProductDetailsComponent?pro ductID=035750#> [extrait le 2007-10-30]

## Description

La présente demande concerne une composition, particulièrement une composition pharmaceutique comprenant au moins un composé de formule 1 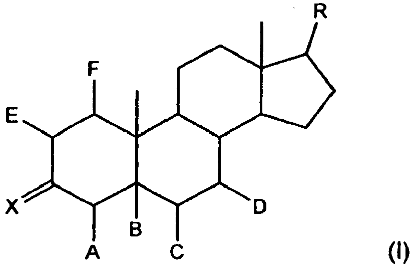 dans laquelle X représente un groupement =N-OH,
R représente un groupement choisi parmi 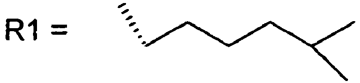 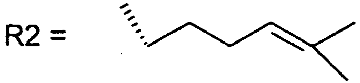 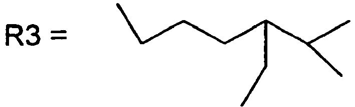 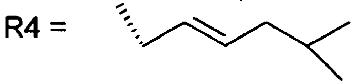 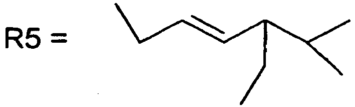 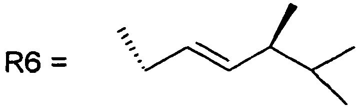
A représente un atome d'hydrogène ou ensemble avec B une liaison carbone-carbone
B représente un atome d'hydrogène, un groupement hydroxy ou ensemble avec A une liaison carbone-carbone,
C représente un atome d'hydrogène ou ensemble avec D une liaison carbone-carbone,
D représente un atome d'hydrogène ou ensemble avec C une liaison carbone-carbone,
E représente un atome d'hydrogène ou ensemble avec F une liaison carbone-carbone,
F représente un atome d'hydrogène ou ensemble avec E une liaison carbone-carbone,
ou l'un de ses esters, particulièrement de l'oxime de cholest-4-èn-3-one.

Plus précisément, l'invention concerne une composition, particulièrement une composition pharmaceutique, administrable oralement contenant un composé tel que décrit précédemment et au moins une huile choisie parmi l'huile de sésame, l'huile d'olive, l'huile de soja, l'huile de coton ou un mélange de triglycérides à chaîne moyenne (comme par exemple l'ESTASAN®, MYGLIOL®), ou un mélange de ces différentes huiles. De préférence, l'huile de sésame sera utilisée.

Les composés de formule I, particulièrement l'oxime de cholest-4-èn-3-one comme par exemple ceux décrits dans la demande internationale WO 2004/082581, sont généralement très insolubles en milieu aqueux. Avec de tels principes actifs, des difficultés apparaissent lors de la mise au point de formulations chimiquement et physiquement stables.

Or ces composés sont de potentiels composés cytoprotecteurs et sont potentiellement utilisables comme médicament, particulièrement comme médicaments utilisables dans le traitement de maladies neurodégénératives.

On comprend donc qu'il existe un réel besoin d'une composition, particulièrement d'une composition pharmaceutique, administrable oralement contenant au moins un composé de formule I, particulièrement de l'oxime de cholest-4-èn-3-one, solubilisé ou en suspension, de telle sorte que le composé soit absorbé au niveau gastro-intestinal.

La composition pharmaceutique selon l'invention a été élaborée en fonction des critères suivants :
- stabilité chimique importante dans le temps des composés selon l'invention ;
- concentration en actif en solution/suspension la plus élevée possible ;
- solution huileuse simple et peu coûteuse en production industrielle ;
- administration possible par une sonde entérale ;
- goût acceptable ou masquable par des arômes classiques ;
- administration possible à des enfants ou des nourrissons ;
- viscosité contrôlée permettant le remplissage de formes pharmaceutiques orales solides.

Par «composition pharmaceutique», on entend dans la présente invention, une composition dont les composants sont pharmaceutiquement acceptables. Par exemple, lorsqu'on envisage une administration orale, les composants sont appropriés ou acceptables pour une administration orale.

Les composés de formule I, particulièrement l'oxime de cholest-4-èn-3-one étant faiblement solubles dans l'eau, une étude a été menée par la Demanderesse afin de déterminer des excipients permettant leur solubilisation ou leur mise en suspension tout en respectant les critères listés ci-dessus.

La Demanderesse a découvert que les produits décrits dans cette demande présentent un très bon comportement avec l'huile de sésame, l'huile d'olive, l'huile de soja, l'huile de coton ou un mélange de triglycérides à chaîne moyenne (ESTASAN®, MYGLIOL® ou équivalent) qui donnent de façon inattendue des résultats bien meilleurs qu'en présence d'autres huiles testées, au point de pouvoir préparer une solution suffisamment concentrée qui autorise une préparation pharmaceutique présentant les avantages suivants :
- stabilité chimique optimale ;
- concentration en actif solubilisé élevée ;
- solution huileuse simple et peu coûteuse en production industrielle;
- utilisation possible pour une administration par une sonde entérale ;
- goût acceptable ;
- adaptation idéale pour une forme pédiatrique.

D'autre part ces propriétés permettent aussi la préparation de solution ou suspension qui peuvent être présentées sous forme de gélules molles ou dures.

La charge par capsule peut être importante ce qui minimise le nombre de capsules par prise journalière.

Une solubilisation et mise en suspension optimale du principe actif ont été obtenues lorsque la composition comprend comme principe actif au moins un composé de formule I, particulièrement l'oxime de la cholest-4-èn-3-one et une huile choisie parmi les huiles suivantes : l'huile de sésame, l'huile d'olive, l'huile de soja, l'huile de coton, le mélange de triglycérides à chaîne moyenne commercial ESTASAN®, MYGLIOL® ou équivalent.

Ainsi, dans son premier objet, l'invention concerne une composition, particulièrement une composition pharmaceutique, caractérisée en ce qu'elle comprend au moins:
+ un composé de formule I 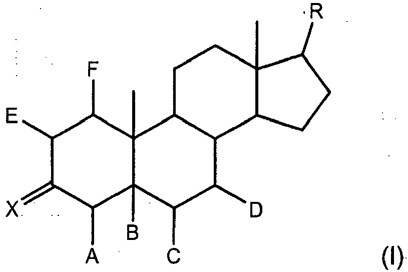
dans laquelle X représente un groupement =N-OH,
R représente un groupement choisi parmi 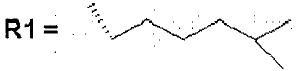 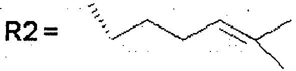 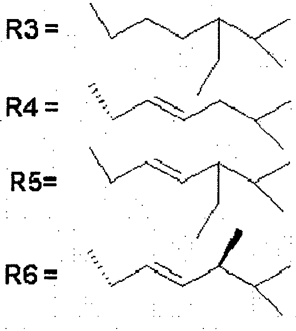
A représente un atome d'hydrogène ou ensemble avec B une liaison carbone-carbone
B représente un atome d'hydrogène, un groupement hydroxy ou ensemble avec A une liaison carbone-carbone,
C représente un atome d'hydrogène ou ensemble avec D une liaison carbone-carbone,
D représente un atome d'hydrogène ou ensemble avec C une liaison carbone-carbone,
E représente un atome d'hydrogène ou ensemble avec F une liaison carbone-carbone,
F représente un atome d'hydrogène ou ensemble avec E une liaison carbone-carbone,
ou l'un de ses esters, et
+ une huile choisie parmi l'huile de sésame, l'huile d'olive, l'huile de soja, l'huile de coton, ou un mélange de triglycérides à chaîne moyenne (ESTASAN®, MYGLIOL® ou équivalent) ou un mélange de ces différentes huiles.

De façon préférée, l'huile utilisée est choisie parmi l'huile de sésame, l'huile d'olive ou l'huile de soja, de préférence l'huile de sésame.

Préférentiellement selon l'invention, le composé de formule I peut être choisi parmi l'oxime de la cholest-4-èn-3-one, l'oxime de cholestan-3-one, l'oxime de cholest-1,4-dièn-3-one, très préférentiellement l'oxime de la cholest-4-èn-3-one,
ou l'un de leurs esters.

Ces composés sont décrits dans la demande internationale publiée le 30 septembre 2004 sous le numéro WO 2004/082581.

Selon l'invention, le composé peut être présent dans la composition pharmaceutique soit sous forme dissoute, pouvant être utilisée notamment dans des sondes entérales ou en forme pédiatrique, il s'agit alors d'une solution, soit sous forme de suspension comprenant de l'actif solubilisé (concentration saturante) et de l'actif sous forme solide, permettant de minimiser le volume de la forme pharmaceutique pour une dose donnée en obtenant ainsi une meilleure acceptation par le patient.

Dans cette composition, le composé est avantageusement présent à des doses physiologiquement efficaces ou représentant un sous multiple de la dose efficace.

Selon l'invention, le composé peut être présent dans la composition en une quantité allant de 10 à 200 mg/ml en solution, préférentiellement de 25 à 150 mg/ml), ou en une quantité allant de 30 à 500 mg/ml en suspension, préférentiellement de 50 à 400 mg/ml.

La composition selon la présente invention peut être administrée par voie orale sous une forme galénique appropriée telle qu'une capsule en gélatine dure ou une capsule molle ou encore une solution buvable ou une suspension buvable de viscosité plus ou moins importante.

La composition selon la présente invention peut être utilisée chez les mammifères, plus précisément chez l'homme.

Les compositions utilisées par voie orale peuvent se présenter sous forme de gélules ou autre forme solide pouvant comporter une phase lipidique, de solution buvable ou de suspension buvable, ou ingérable.

Les quantités des différents constituants des compositions selon l'invention sont celles classiquement utilisées dans les domaines considérés.

De façon connue, la composition de l'invention peut contenir également des adjuvants habituels dans les domaines considérés, tels que par exemple les conservateurs, les antioxydants, les pigments et les matières colorantes, les épaississants, les tensioactifs, les arômes, les édulcorants, les agents stabilisant les particules d'actifs.

Les quantités de ces différents adjuvants sont celles classiquement utilisées dans les domaines considérés, et par exemple de 0,0001 % à 10 % du poids total de la composition. Ces adjuvants sont introduits dans la phase grasse.

Les exemples suivants illustrent l'invention sans pour autant la limiter.

### Exemple 1 : comparaison de la stabilité de l'oxime de cholest-4-èn-3-one dans différentes huiles

La stabilité de l'oxime de cholest-4-èn-3-one a été testée dans 12 huiles différentes. Le composé est ajouté à l'huile à une concentration de 15 mg/ml sous forme de poudre micronisée, et le mélange est placé 15 jours dans une étuve à 60°C. La quantité d'impureté est mesurée dans la suspension, diluée de façon à se trouver à une concentration de composés à 2,510⁻⁴M, par HPLC UV à 255 nm. L'impureté majeure obtenue est la cholesténone. D'autres impuretés peuvent aussi apparaître, certaines connues (et dont on sait qu'elles sont stables), alors que d'autres impuretés apparues sont inconnues, et dont on ne connaît donc pas le potentiel de toxicité ou de stabilité.

Les stabilités sont indiquées dans le tableau ci-dessous. Le produit est considéré comme très stable (++, très stable) dans une huile donnée si après les 15 jours d'incubation dans l'étuve à 60°C, le pourcentage de l'aire totale sous la courbe du chromatogramme obtenu mesuré correspondant à des pics de produits de dégradation de l'oxime de cholest-4-èn-3-one représente moins de 1%, et sont des produits connus, stables. Le produit est considéré comme stable (+, stable) dans une huile si après les 15 jours passés dans l'étuve à 60°C, le pourcentage de l'aire totale sous la courbe du chromatogramme obtenu mesuré correspondant à des pics de produits de dégradation de l'oxime de cholest-4-èn-3-one représente moins de 1 %, mais correspond à des produits de dégradation inconnus. Le produit est considéré comme instable (-, instable) dans une huile si après les 15 jours passés dans l'étuve à 60°C, le pourcentage de l'aire totale sous la courbe du chromatogramme obtenu mesuré correspondant à des pics de produits de dégradation de l'oxime de cholest-4-èn-3-one représente plus de 1%.

| | stabilité |
|---|---|
| Huile de sésame | ++ |
| Huile d'olive | ++ |
| Huile de soja | ++ |
| Huile de coton | ++ |
| Mélange de triglycérides à chaîne moyenne (ESTASAN®, MYGLIOL®) | ++ |
| Huile de ricin | + |
| Huile d'arachide | + |
| Huile de colza | + |
| Huile de maïs | - |
| Huile d'amande | - |
| Huile de tournesol | - |
| Acide oléique | - |

| | |
|---|---|
| (-) : instabilité majeure du produit dans l'huile testée, avec libération de plus de 1% de produits de dégradation, en 2 semaines à 60°C. (+) : composé stable dans l'huile testée, avec libération de moins de 1% de produits de dégradation inconnus, en deux semaines à 60°C. (++) : composé très stable dans l'huile testée, avec libération de moins de 1% de produits de dégradation connus, stables en deux semaines à 60°C. | |

Conclusion : les huiles préférées sont celles qui dégradent le moins l'oxime de cholest-4-èn-3-one dans les mélanges au cours du temps. Il s'agit de l'huile de sésame, l'huile d'olive, l'huile de soja, l'huile de coton, le mélange de triglycérides à chaîne moyenne (ESTASAN®, MYGLIOL®), l'huile de ricin, l'huile d'arachide, et l'huile de colza. L'huile de maïs, l'huile d'amande, l'huile de tournesol et l'acide oléique donnent quant à eux des résultats insatisfaisants. Parmi les huiles avec lesquelles on obtient le moins de produits de dégradation, on préfère celles se dégradant en des composés identifiés et dont on sait qu'ils sont stables dans le temps (résultats non montrés). Dans ce classement, les huiles les meilleures sont l'huile de sésame, l'huile d'olive, l'huile de soja, l'huile de coton et le mélange de triglycérides à chaîne moyenne (ESTASAN®, MYGLIOL®). Parmi celles-ci, l'huile de sésame, l'huile d'olive et l'huile de soja sont préférées, car elles présentent le goût le plus acceptable parmi l'ensemble des 5 huiles préférées. L'huile de sésame est l'huile préférée parmi ces trois huiles car elle permet la stabilité la plus élevée de l'oxime de cholest-4-èn-3-one.

### Exemple 2 : comparaison de la solubilité de l'oxime de cholest-4-èn-3-one dans l'eau et dans différentes huiles.

Des essais ont été conduits afin de mettre en évidence la solubilité maximale de l'oxime de cholest-4-èn-3-one dans l'eau et dans les différentes huiles retenues à la suite du test de stabilité (exemple 1).

| | solubilité maximale (mg/ml) |
|---|---|
| Eau | 0,001 |
| Huile de sésame | 35 |
| Huile d'olive | 40 |
| Soja | 30 |
| Coton | 28 |

| | |
|---|---|
| Mélange de triglycérides à chaîne moyenne (ESTASAN®, MYGLIOL®) | 45 |
| Ricin | <15 |
| Arachide | <15 |
| Colza | <15 |

La meilleure solubilité a été obtenue avec le mélange de triglycérides à chaîne moyenne (ESTASAN®, MYGLIOL®), suivi des huiles d'olive, de sésame et de soja.

### Exemple 3 : la composition suivante est réalisée

| Composant : | % poids |
|---|---|
| Oxime de cholest-4-èn-3-one | 43,4 |
| Huile de sésame raffinée* | 50,8 |
| Adjuvant tensioactif : lécithine de soja* | 1,0 |
| Adjuvant épaississant : mélange d'huiles végétales hydrogénées NF type II** | 4,8 |

| | |
|---|---|
| * selon les normes de la pharmacopée européenne ** selon les normes de la pharmacopée des Etats Unis | |

La composition est encapsulée, de préférence dans des capsules molles, et se présente donc sous forme solide. L'avantage de cette composition est la quantité importante de composé mis en suspension par capsule, ce qui limite le volume de prise. Il n'est pas besoin d'aromatiser le mélange avant encapsulation, puisque le mélange a un goût acceptable.

Des capsules contenant cette composition (165 mg d'oxime de cholest-4-èn-3-one par capsule) ont été stockées dans des bouteilles plastiques fermées par un bouchon plastique non hermétique (50 capsules par bouteille) à 40°C et 75% d'humidité pendant 1 et 2 mois. Après 1 et 2 mois, la composition est stable puisque le niveau d'impuretés nouvellement apparu est inférieur à 1% total.

### Exemple 4 : la composition suivante est réalisée :

Le composé oxime de cholest-4-èn-3-one est dissout à 30 mg/ml dans de l'huile de sésame. La composition ainsi obtenue se présente sous forme d'une solution.

Cette composition liquide est particulièrement intéressante car elle est généralement plus facile à avaler qu'une capsule, la dose peut être facilement adaptée en pipetant le volume nécessaire. Le mélange ne nécessite ni aromatisation, ni ajout d'édulcorant, car le goût est acceptable. Cette composition présente l'avantage d'être simple et peu onéreuse. De plus, elle correspond aux critères pharmaceutiques décrits pour une forme pédiatrique et peut donc être utilisé dans ces populations ou pour des populations plus âgées ne pouvant être nourries que par sonde entérale.

La composition est particulièrement stable après au moins 2 semaines à 40°C puisque le niveau d'impuretés nouvellement apparu est inférieur à 1 % total. Elle est stable également à 60°C.

## Revendications

1. Composition, particulièrement composition pharmaceutique, **caractérisée en ce qu'**elle comprend au moins :
+ un composé de formule I 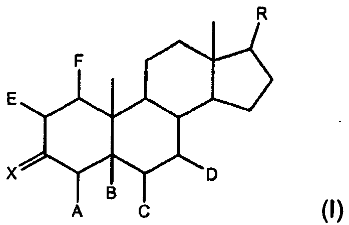
dans laquelle X représente un groupement =N-OH,
R représente un groupement choisi parmi 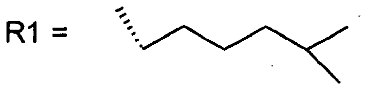 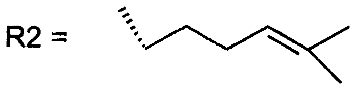 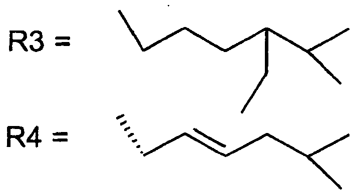 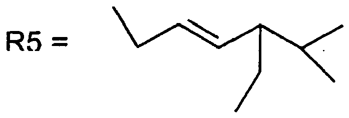 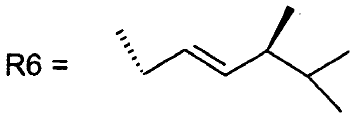
A représente un atome d'hydrogène ou ensemble avec B une liaison carbone-carbone
B représente un atome d'hydrogène, un groupement hydroxy ou ensemble avec A une liaison carbone-carbone,
C représente un atome d'hydrogène ou ensemble avec D une liaison carbone-carbone,
D représente un atome d'hydrogène ou ensemble avec C une liaison carbone-carbone,
E représente un atome d'hydrogène ou ensemble avec F une liaison carbone-carbone,
F représente un atome d'hydrogène ou ensemble avec E une liaison carbone-carbone,
ou l'un de ses esters, et
+ une huile choisie parmi l'huile de sésame, l'huile d'olive, l'huile de soja, l'huile de coton, ou un mélange de triglycérides à chaîne moyenne (ESTASAN®, MYGLIOL®) ou un mélange de ces différentes huiles.

2. Composition selon la revendication 1, **caractérisée en ce que** l'huile est choisie parmi l'huile de sésame, l'huile d'olive et l'huile de soja.

3. Composition selon l'une des revendications 1 ou 2, **caractérisée en ce que** l'huile est l'huile de sésame.

4. Composition selon l'une des revendications 1 à 3, **caractérisée en ce que** le composé de formule I est choisi parmi l'oxime de la cholest-4-èn-3-one, l'oxime de cholestan-3-one, l'oxime de cholest-1,4-dièn-3-one.

5. Composition selon l'une des revendications 1 à 4, **caractérisée en ce que** le composé de formule I est choisi l'oxime de la cholest-4-èn-3-one, ou l'un de leurs esters.

6. Composition selon l'une des revendications 1 à 5, **caractérisée en ce que** le composé est présent dans la composition sous forme de solution en une quantité allant de 10 à 200 mg/ml.

7. Composition selon l'une des revendications 1 à 6, **caractérisée en ce que** le composé est présent dans la composition sous forme de suspension en une quantité allant de 30 à 500 mg/ml.

8. Composition comprenant le composé oxime de cholest-4-èn-3-one et l'huile de sésame.

9. Composition comprenant le composé oxime de cholest-4-èn-3-one, l'huile de sésame, la lécithine de soja et un mélange d'huiles végétales hydrogénées.

## Patentansprüche

1. Zusammensetzung, insbesondere eine pharmazeutische Zusammensetzung, **dadurch gekennzeichnet, daß** sie mindestens das Folgendes umfasst:
+ eine Verbindung gemäß Formel I 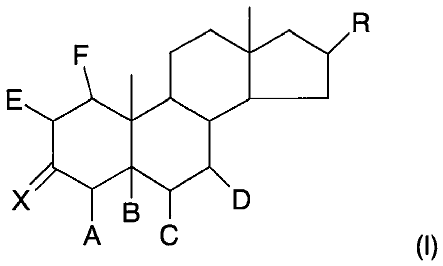
bei der X eine Gruppe =N-OH repräsentiert,
R eine Gruppe repräsentiert, die ausgewählt ist aus 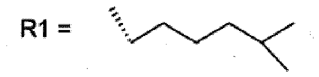 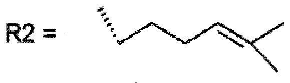 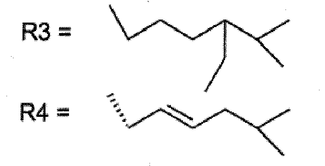 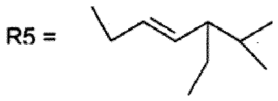 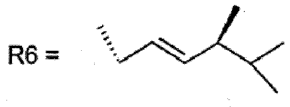
A ein Wasserstoffatom oder zusammen mit B eine Kohlenstoff-Kohlenstoff-Bindung repräsentiert,
B ein Wasserstoffatom, eine Hydroxygruppe oder zusammen mit A eine Kohlenstoff-Kohlenstoff-Bindung repräsentiert,
C ein Wasserstoffatom oder zusammen mit D eine Kohlenstoff-Kohlenstoff-Bindung repräsentiert,
D ein Wasserstoffatom oder zusammen mit C eine Kohlenstoff-Kohlenstoff-Bindung repräsentiert,
E ein Wasserstoffatom oder zusammen mit F eine Kohlenstoff-Kohlenstoff-Bindung repräsentiert,
F ein Wasserstoffatom oder zusammen mit E eine Kohlenstoff-Kohlenstoff-Bindung repräsentiert,
oder eines seiner Ester, und
+ ein Öl, das ausgewählt ist aus Sesamöl, Olivenöl, Sojaöl, Baumwollsamenöl, oder eine Mischung aus mittelkettigen Triglyceriden (ESTASAN®, MYGLIOL®) oder eine Mischung aus diesen verschiedenen Ölen.

2. Zusammensetzung nach Anspruch 1, **dadurch gekennzeichnet, dass** das Öl aus Sesamöl, Olivenöl und Sojaöl ausgewählt ist.

3. Zusammensetzung nach einem der Ansprüche 1 oder 2, **dadurch gekennzeichnet, dass** das Öl Sesamöl ist.

4. Zusammensetzung nach einem der Ansprüche 1 bis 3, **dadurch gekennzeichnet, dass** die Verbindung gemäß Formel I ausgewählt ist aus dem Oxim von Cholest-4-en-3-on, dem Oxim von Cholestan-3-on, dem Oxim von Cholest-1,4-dien-3-on.

5. Zusammensetzung nach einem der Ansprüche 1 bis 4, **dadurch gekennzeichnet, dass** die Verbindung gemäß Formel I ausgewählt ist aus dem Oxim von Cholest-4-en-3-on oder eines seiner Ester.

6. Zusammensetzung nach einem der Ansprüche 1 bis 5, **dadurch gekennzeichnet, dass** die Verbindung als Lösung in einer Menge von 10 bis 200 mg/ml in der Zusammensetzung vorhanden ist.

7. Zusammensetzung nach einem der Ansprüche 1 bis 6, **dadurch gekennzeichnet, dass** die Verbindung als Suspension in einer Menge von 30 bis 500 mg/ml in der Zusammensetzung vorhanden ist.

8. Zusammensetzung, die die Verbindung Oxim von Cholest-4-en-3-on und Sesamöl umfasst.

9. Zusammensetzung, die die Verbindung Oxim von Cholest-4-en-3-on, Sesamöl, Sojalezithin und eine Mischung aus hydrierten Pflanzenölen umfasst.

## Claims

1. Composition, particularly a pharmaceutical composition, **characterized in that** it comprises at least:
+ one compound of Formula I 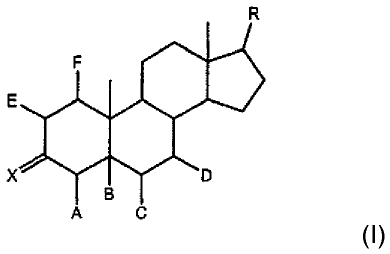
in which X represents an =N-OH group,
R represents a group chosen from 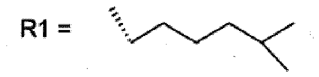 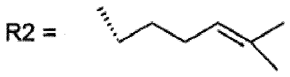 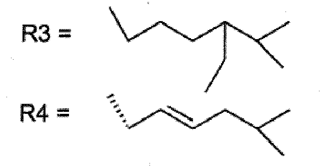 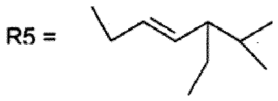 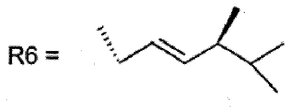
A represents a hydrogen atom or, together with B, a carbon-carbon bond,
B represents a hydrogen atom, a hydroxy group or, together with A, a carbon-carbon bond,
C represents a hydrogen atom or, together with D, a carbon-carbon bond,
D represents a hydrogen atom or, together with C, a carbon-carbon bond,
E represents a hydrogen atom or, together with F, a carbon-carbon bond,
F represents a hydrogen atom or, together with E, a carbon-carbon bond,
or an ester thereof, and
+ an oil chosen from sesame oil, olive oil, soya oil, cotton seed oil, or a mixture of medium-chain triglycerides (ESTASAN®, MYGLIOL®) or a mixture of these different oils.

2. Composition according to claim 1, **characterized in that** the oil is chosen from sesame oil, olive oil and soya oil.

3. Composition according to one of claims 1 or 2, **characterized in that** the oil is sesame oil.

4. Composition according to one of claims 1 to 3, **characterized in that** the compound of Formula I is chosen from cholest-4-en-3-one oxime, cholestan-3-one oxime, cholest-1,4-dien-3-one oxime.

5. Composition according to one of claims 1 to 4, **characterized in that** the compound of formula I is chosen from cholest-4-en-3-one oxime, or an ester thereof.

6. Composition according to one of claims 1 to 5, **characterized in that** the compound is present in the composition in the form of a solution in a quantity ranging from 10 to 200 mg/ml.

7. Composition according to one of claims 1 to 6, **characterized in that** compound is present in the composition in the form of a suspension in a quantity ranging from 30 to 500 mg/ml.

8. Composition comprising the cholest-4-en-3-one oxime compound and sesame oil.

9. Composition comprising the cholest-4-en-3-one oxime compound, sesame oil, soya lecithin and a mixture of hydrogenated vegetable oils.
